# EUROPEAN PATENT SPECIFICATION

(11) **EP 0 268 663 B1**
(45) Date of publication and mention of the grant of the patent: **27.12.1995**
(21) Application number: 87904015.2
(22) Date of filing: 12.06.1987
(51) Int. Cl.: C07K 7/06, C07K 7/08, C07K 14/47, A61K 38/01, A61K 38/04, A61K 38/08, A61K 38/16, A61K 7/26, C12P 21/06

(54) **PHOSPHOPEPTIDES**
PHOSPHOPEPTIDE
PHOSPHOPEPTIDES

(30) Priority: 12.06.1986 AU 63/85
(43) Date of publication of application: 01.06.1988
(73) Proprietor: THE UNIVERSITY OF MELBOURNE, Parkville, VIC 3065 (AU); VICTORIAN DAIRY INDUSTRY AUTHORITY, Hawthorn Victoria 3122 (AU)
(72) Inventor: REYNOLDS, Eric, Charles, North Balwyn, VIC 3103 (AU)
(74) Representative: Allen, Oliver John Richard
(86) International application number: AU8700172
(87) International publication number: WO8707615

(56) References cited:
- WO-A-82/03008
- AU-A- 1 956 783
- AU-B- 3 961 978
- DE-A- 3 320 175
- US-A- 4 358 465
- US-A- 4 361 587
- US-A- 4 495 176
- J Dairy Res 44 (1977) 373-376
- Proceedings of the 7th American Peptide Symposium : Syntheses, Sructure and Function, 1981, pages 65-67, Pierce Chemical Co., Rockford, US; P F Alewood et al.:"Synthesis of casein related peptides"

## Description

This invention relates to phosphopeptides and compositions containing same.

This invention also relates to caries and gingivitis inhibition.

US Patent No. 4 495 176 discloses enzymatic hydrolysis of phosphocaseinates to yield oligophosphopeptides which may be useful as alimentary products. West in Journal of Dairy Research 44(1977) p373-376 discusses the digestion of casein with trypsin, and in particular following pre-treatment with cyanogen bromide. Alewood et al, Proc. of the 7th Amer. Peptide Symp: Synthesis, Structure and Function 1981 p65-67, discloses a synthetic octaphosphopeptide derived from casein. International publication No. WO82/03008 discusses the use of phosphoproteins for example casein or phosvitin or polyphosphoserine for the inhibition of caries formation.

The present invention provides a composition having anticariogenic activity comprising one or more phosphopeptides selected from: 
Glu-Met-Glu-Ala-Glu-Pse-Ile-Pse-Pse-Pse-Glu-Glu-Ile-
Val-Pro-Asn-Pse-Val-Glu-Gln-Lys;
Glu-Leu-Glu-Glu-Leu-Asn-Val-Pro-Gly-Glu-Ile-Val-Glu-
Pse- Leu-Pse-Pse-Pse-Glu-Glu-Ser-Ile-Thr-Arg;
Asn-Thr-Met-Glu-His-Val-Pse-Pse-Pse-Glu-Glu-Ser-Ile-
Ile- Pse-Gln-Glu-Thr-Tyr-Lys;
Asn-Ala-Asn-Glu-Glu-Glu-Tyr-Ser-Ile-Gly-Pse-Pse-Pse-
Glu-Glu-Pse-Ala-Glu-Val-Ala-Thr-Glu-Glu-Val-Lys; and
Glu-Gln-Leu-Pse-Pth-Pse-Glu-Glu-Asn-Ser-Lys
in the form of an acid or a salt.

The phospeptide is preferably in substantially pure form.

The phosphopeptides of the present invention or their salts may have utility in the treatment or inhibition of (i) dental diseases such as caries, gingivitis and periodontal disease, (ii) rarefying bone diseases such as osteoporosis and osteomalacia and (iii) diseases relating to malabsorption of minerals.

Accordingly, the present invention provides a composition comprising a peptide or a salt thereof in accordance with this invention and a physiologically acceptable diluent.

The composition may be in the form of a pharmaceutical composition.

The composition may be orally ingestible.

A mixture of phosphopeptides and/or their salts may be used in the composition.

The phosphopeptide or mixture of phosphopeptides is preferably substantially pure at least to the extent of not containing unpalatable impurities.

The amino acid symbols are as follows: Pse-phosphoserine, Ser-Serine, Pth-phosphothreonine, Thr-threonine, Glu-glutamate, Asp-aspartate, Ala-alanine, Asn-asparagine, Gln-glutamine, Gly-glycine, Arg-arginine, His-histidine, Ile-isoleucine, Leu-leucine, Lys-lysine, Met-methionine, Pro-proline, Tyr-tyrosine, Val-valine.

The phosphopeptide may be made synthetically by chemical synthesis or genetic engineering or can be extracted from naturally occurring material.

Because of cost considerations it is currently more economic to extract the phosphopeptide from casein and in particular from alpha-s casein or beta-casein. Phosvitin may also be used as a source of the peptide. Further, phosphoproteins in cereals, nuts and vegetables particularly in bran husks or sheaths may be used to produce the peptide above. In particular, rice, wheat, oat, barley or rye brans. Soybean and meat contain phosphoproteins which may be of use in obtaining the peptide above.

Casein and in particular alpha-s casein or beta-casein or salts thereof such as sodium caseinate contain polypeptides which can be cleaved to simpler peptides. Such cleavage may be effected by digestion, such digestion may be chemical or proteolytic.

It is presently preferred to digest casein with one of trypsin, pepsin, chymotrypsin, papain, theremolysin or pronase. Of these, trypsin is preferred.

The digested casein can be fractioned into the desired peptides and other peptides. The presence of said other peptides is not deleterious to efficacy, however, certain of said other peptides have objectionable taste and accordingly if any of said other peptides are to be included it is preferable to remove those having objectionable taste. In general, those of said other peptides having objectionable taste seem to be hydrophobic.

The following peptides have been found to have objectionable taste:-
1. Glu-Val-Leu-Asn
2. Asn-Glu-Asn-Leu-Leu
3. Ala-Pro-Phe-Pro-Gln-Val-Phe-Gly
4. Leu-Arg-Phe
5. Phe-Phe-Val-Ala-Pro-Phe-Pro-Gln-Val-Phe-Gly-Lys
6. Leu-Arg-Leu
7. Phe-Tyr-Pro-Glu-Leu-Phe
(Glu-glutamate; Val-valine; Leu-leucine; Asn-asparagine; Ala-alanine; Pro-proline; Phe-phenylalanine; Gln-glutamine; Gly-glycine; Arg-Arginine; Lys-lysine; Tyr-tyrosine.)

Preferably the peptide is one exhibiting a reduction in hydroxy apatite dissolution rate of at least 15% under the test conditions defined herein.

Preferably the peptide is one exhibiting a reduction in hydroxy apatite dissolution rate of at least 26% under the test conditions defined herein.

Preferably, the peptide is one exhibiting a reduction in hydroxy apatite dissolution rate of at least 30% under the test conditions defined herein.

Preferably, the peptide is one exhibiting a reduction in hydroxy apatite dissolution rate of at least 32% under the test conditions defined herein.

Preferably, the peptide is present as 0.01 to 10% by weight.

Preferably, the peptide is present as 0.01 to 5% by weight.

Preferably , the peptide is present as 0.01 to 2% by weight.

The composition of this invention may be in the form of a comestible such as foodstuff or confectionery, dentifrice, tablet or comprise a pharmacologically acceptable vehicle or solution of suspension for topical application to the teeth or gingival tissues or a mouthwash. Other modes of administering the peptide would be acceptable if physiologically or pharmacologically acceptable.

Of particular interest as compositions are chewing gum, breakfast foods, ice-cream and other frozen confectionery, confectionery, sweets and cakes as these are all known as caries problem materials. Similar considerations apply to other potentially cariogenic food components.

Also of particular interest are dentifrices, mouthwashes and preparations for topical application to teeth and gingival tissue and enteric capsules for the treatment of bone disorders and mineral malabsorption.

Also of interest is the compositions in accordance with this invention for dental treatment of cavities. In this respect, there appears to be evidence of remineralization of incipient lesions which are considered to be a pre-cavity condition. However, there is also evidence to indicate that application of compositions in accordance with this invention to the surfaces of actual cavities and to surfaces of teeth produced by removal of decay material from actual cavities or by fracture is beneficial.

Since a topical application of a composition in accordance with this invention which is an aqueous solution to surfaces of actual cavities or surfaces of teeth produced by removal of decay material from actual cavities or by fracture is unlikely to have long term effect, we have further sought to provide compositions which might have the desired long term effect.

Accordingly, the present invent ion also provides a composition in accordance with this invention and adapted to remain in contact with a tooth surface over a prolonged period. The invention also provides methods and means for maintaining compositions in accordance with this invention in contact with a tooth surface over a prolonged period.

In this last respect a prolonged period should be interpreted in accordance with the effect desired and the time taken to achieve sufficient of that effect to be of value. However, in some instances that prolonged period may be as short as one day but is more preferably a period of weeks or months.

In one instance a tooth cavity is coated with a composition in accordance with this invention and the cavity is closed to restrict escape of the composition. Such closure may be effected by capping or use of dental cavity filling compositions.

In another instance the composition is so formulated as to be adapted to remain in place for a prolonged period. In this instance the composition of the invention may form part of a dental filling composition.

Accordingly, the present invention also provides a dental filling composition comprising a phosphopeptide as defined above and a carrier therefor adapted to adhere the composition to a tooth surface.

Such a dental filling composition may contain dental filling materials known per se including amalgams and settable polymers.

Of particular interest are dental filling compositions which contain calcium. The calcium is desirably in the form of calcium phosphate or hydroxyapatite.

The phosphopeptides for use in the invention can be extracted in a number of ways but the use of a fractionation technique is generally preferred.

The phosphopeptides can be extracted by fractionation based on molecular size or charge characteristics. Due to the unique negative charge density and divalent metal ion sequestering ability of the peptides, the preferred fractionation procedure is anion exchange chromatography or selective precipitation or a combination of both.

The following procedure illustrates one mode of extraction.

### Extraction Procedure I.

An example of the phosphopeptides are those produced by a tryptic digestion of bovine milk casein. The digestion of whole sodium caseinate or fractions (alpha-S or beta) produced by selective precipitation (Zittle, C.A. and Custer J.H.; J. Dairy Sci 46L 1183-1189, 1963) is carried out using a protein: trypsin ratio of 50:1 in 20 mM Tris HCl pH 8.0, 2.5mM NaCl at 37°C for 1h. The peptides were fractionated using a Pharmacia FPLC system with a Mono Q HR 5/5 column and eluted with a NaCl gradient; Buffer A 20mM Tris HCl pH 8.0, 2.5mM NaCl; Buffer B 20 mM Tris HCl pH 8.0, 500mM NaCl, gradient 0-100% Buffer B/30 min; flow rate 1ml/min. Fraction were washed and concentrated using an Amicon Ultrafiltration Cell with a UMO5 filter. The peptides were identified using a Water Associates PICO-TAG amino acid analysis system using phonylisothiocyanate amino acid derivatisation. Phosphate was measured by the method of Itaya and Ui (Clin, Chim. Acta. 14:361-366, 1960). The peptides were sequenced (after the removal of phosphate by alkaline phosphatase) using manual Edman degradation and the resulting PTH-amino acids identified using reverse phase HPLC on a Zorbax ODS column 25x0.46 cm (DuPont).

The following phosphopeptides were individually obtained from a tryptic digestion of sodium caseinate using the above procedure.

T1.Glu-Met-Glu-Ala-Glu-Pse-Ile-Pse-Pse-Pse-Glu-Glu-Ile-Val-
Pro-Asn-Pse-Val-Glu-Gln-Lys.
T2.Glu-Leu-Glu-Glu-Leu-Asn-Val-Pro-Gly-Glu-Ile-Val-Glu-Pse-
Leu-Pse-Pse-Pse-Glu-Glu-Ser-Ile-Thr-Arg.
T3.Asn-Thr-Met-Glu-His-Val-Pse-Pse-Pse-Glu-Glu-Ser-Ile-Ile-
Pse-Gln-Glu-Thr-Tyr-Lys.
T4.Asn-Ala-Asn-Glu-Glu-Glu-Tyr-Ser-Ile-Gly-Pse-Pse-Pse-Glu-
Glu-Pse-Ala-Glu-Val-Ala-Thr-Glu-Glu-Val-Lys.
T5.Glu-Gln-Leu-Pse-Pth-Pse-Glu-Glu-Asn-Ser-Lys.

In addition the following peptides were also obtained:

T6.Asp-Ile-Gly-Pse-Glu-Pse-Thr-Glu-Asp-Gln-Ala-Met-Glu-Asp-
Ile-Lys.
T7.Val-Pro-Gln-Leu-Gln-Ile-Val-Pro-Asn-Pse-Ala-Glu-Glu-Arg.
T8.Thr-Val-Asp-Met-Glu-Pse-Thr-Glu-Val-Phe-Thr-Lys.
T9.Leu-Pth-Glu-Glu-Lys.

The peptides T1,T6 and T7 were also obtained from a TPCK-tryptic digest of alphaₛ₁-caseinate(comprising alphaₛ₁ and alpahaₛ₀). Peptide T2 was also obtained from a TPCK-tryptic digest of beta-caseinate. Peptides T3, T4, T5, T8 and T9 were also obtained from a TPCK -tryptic digest of alphaₛ₂-caseinate (comprising alphaₛ₂,alphaₛ₃, alphaₛ₄ and alphaₛ₆). The amino acid symbols are as follows: Pse-phosphoserine, Ser- serine, Pth-phosphothreonine, Thr-threonine, Glu- Glutamate, Asp- aspartate, Ala- alanine, Asn- aspargine, Gln- glutamine, Gly- glycine, Arg- arginine, His- histidine, Ile- isoleucine, Leu- leucine, Lys- lysine, Met - methionine, Pro- proline, Tyr- tyrosine, Val- valine.

### Extraction Procedure II

The following procedure illustrates one mode of selective precipitation.

A solution of sodium caseinate was digested with trypsin (50:1, casein:trypsin) for one hour at 37°C with the pH maintained at 8.0 by the addition of NaOH. HCl (0.1M) was than added to the solution at room temperature to pH 4.7 and the resulting precipitate removed. BaCl₂ was added to the supernatant to a level of 0.25% w/v followed by an equal volume of absolute ethanol and the resulting precipitate was removed and dried. The precipitate was dissolved in one tenth the original volume of water (to facilitate dissolution the pH was raised with NaOH) and the solution acidified to pH 3.5 with 1M HCl. An equal volume of acetone was added and the precipitate removed and dried. The precipitate was than redissolved in H₂O and acidified to pH 2.0 by addition of HCl. The resulting precipitate was removed and discarded and the supernatant was adjusted back to pH 3.5 with NaOH and an equal volume of acetone was added. The resulting precipitate was collected, redissolved in water and H₂SO₄ added to precipitate BaSO₄ which was discarded. The supernatant was then dialysed and lyophylised or spray dried. A mixture of 5 phosphopeptides were obtained with this procedure.

The following are the phosphopeptides obtained:-

T1.Glu-Met-Glu-Ala-Glu-Pse-Ile-Pse-Pse-Pse-Glu-Glu-Ile-Val-
Pro-Asn-Pse-Val-Glu-Gln-Lys.
T2.Glu-Leu-Glu-Glu-Leu-Asn-Val-Pro-Gly-Glu-Ile-Val-Glu-Pse-
Leu-Pse-Pse-Pse-Glu-Glu-Ser-Ile-Thr-Arg.
T3.Asn-Thr-Met-Glu-His-Val-Pse-Pse-Pse-Glu-Glu-Ser-Ile-Ile-
Pse-Gln-Glu-Thr-Tyr-Lys.
T4.Asn-Ala-Asn-Glu-Glu-Glu-Tyr-Ser-Ile-Gly-Pse-Pse-Pse-Glu-
Glu-Pse-Ala-Glu-Val-Ala-Thr-Glu-Glu-Val-Lys.
T5.Glu-Gln-Leu-Pse-Pth-Pse-Glu-Asn-Ser-Lys.

The ratio of the phosphopeptides (T1;T2:T3:T4:T5) in the final preparation depends on the starting material and conditions of hydrolysis. Digesting sodium caseinate with TPCK-trypsin yields largely T2 with small amounts of T1, T3 and T4. However, T2 shows greater lability than the other peptides such that more rigorous digestion as occurs with some commercial casein digests yields a preparation containing largely T1 with small amounts of T3 and T4.

If in lieu of sodium caseinate, alpha s1-casein is used for this procedure pure T1 is obtained. With beta-casein as the starting material pure T2 is obtained.

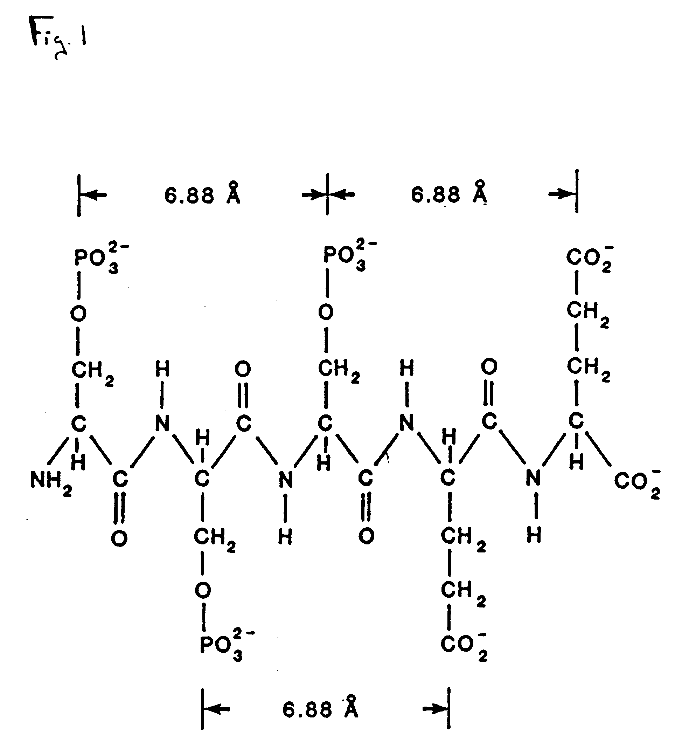

The most common sequences of the active peptides is the pentapeptide Pse-Pse-Pse-Glu-Glu. The spacings of the phosphate and carboxyl groups in a beta-conformation of this pentapeptide are shown in Fig 1.

The 6.88 Angstrom spacings of phosphates and carboxyls allows specific attachment to calcium atoms along the c-axis of hydroxyapatite crystals. This pentapeptide sequence occurs in peptides T1 to T4 and occurs modified in peptide T5 - Pse-Pth-Pse-Glu-Glu following a conservative substitution of phosphothreonine for phosphoserine.

Conservative substitutions within the active sequence would be phosphothreonine and to a lesser extent phosphotryrosine or phosphohistidine for phosphoserine although phosphoserine is preferable. Another possible substitution for phosphoserine would be glutamate or aspartate but again phosphoserine is preferable. A possible substitution for glutamate is aspartate.

The active peptides can sequester calcium phosphate and other salts of divalent metal ions. One mole of T1 binds 16 mole of CaHPO₄ such that a 10mg/ml solution of T1 at pH 7.0 can solubilize 60mM CaHPO₄ producing a metastable supersaturated solution with respect to calcium phosphate species. With chloride as the counter ion one mole of T1 binds only 5 mole of Ca⁺⁺ binding only to serine phosphates. One mole of T1 with about 16 mole of CaHPO₄ bound (M.W. 4883) will henceforth be referred to as calcium phosphate T1. An important chemical feature of calcium phosphate T1 is that above 2% w/v in water the composition is a thixotropic gel. T1-T5 have been shown to be potentially anticariogenic using the following test procedures:

### Test 1. Hydroxyapatite Dissolution Rate Assay.

This test is a modification of a test procedure already described (Reynolds, E.C., Riley, P.F. and Storey, E. Calcif. Tiss Int 34:s52-s56, 1982). The purpose of this test is to determine the affect of the peptides on hydroxyapatite dissolution and in this respect since tooth enamel is largely composed of hydroxyapatite it is believed that useful comparisons can be made.

Double distilled, deionized water (18 mega ohms/cm) was used throughout. Analytical reagent grade chemicals were obtained from Ajax Chemicals, Australia. Hydroxyapatite-spheriodal was purchased from BDH. A chromatography column containing 0.1 g of hydroxyapatite beads was used for the demineralisation assay. Tris 5mM, pH 8.3 containing 50mM NaCl was used as the column buffer at 20°C and was pumped through the column at a rate of 0.1ml/min. A peptide solution 0.1mg/ml of buffer was applied to the column and 0.2ml fractions were collected before and after peptide application and assayed for total calcium, phosphate and peptide. From these values a rate of dissolution (nmol calcium or phosphate per min) for each 0.2ml fraction was obtained.

Phosphopeptides T1-T5 all decreased hydroxyapatite dissolution rate by about 32%.

Phosphopeptides T6-T9 were found to be much less effective.

Fluoride plus phosphopeptide T1 gave a combined reduction in hydroxyapatite dissolution (40% reduction). The phosphopeptide T1 caused a 50% greater retention of fluoride in the hydroxyapatite column.

This work shows that these phosphopeptides bind to hydroxyapatite end reduce the minerals dissolution rate and enhance the retention of fluoride in the crystal matrix. The reduction in hydroxyapatite dissolution was related to the phosphoserine content and spacings within the peptides.

### Test 2. Intra-Oral Caries Test.

The anticariogenicity of phosphopeptide T1 was determined using a modification of the intra-oral caries test of Koulourides and Ostrom (Caries Res. 10:442-482, 1976). Enamel slabs were inset in a removable intra-oral appliance to simulate an approximal area. This was done on both sides of the removable appliance (left and right). The appliance was worn to allow plaque accumulation in the simulated approximal areas. Eight times a day the appliance was removed and placed in a solution at 37°C. The solution was 2% w/v sucrose, 2% w/v glucose, 140 mM KCl, 20mM NaCl at pH 7.0. Twice a day the right side enamel slabs received exposure to a solution containing 1.8% w/v calcium phosphate T1 in 140 mM KCl, 20 mM NaCl at pH 7.0, while the left side received only the salt solution. At the completion of the experiment the enamel slabs were removed, sectioned and subjected to microradiography and microhardnass testing. The microradiography showed that the slabs exposed to the sugar-salt solution (left-side) had sub-surface, caries-like lesions. However, the slabs exposed to the sugar-salt solution and the peptide T1 solution twice a day showed no caries-like changes. The results were confirmed by microhardness analysis. Plaque was also taken from both sides of the appliance and analysed for calcium phosphate, serine phosphate and peptide T1 using a competitive, quantitative, enzyme-linked immunosorbent assay (ELISA) utilising monoclonal antipeptide T1 antibodies.

This showed that the plaque on the right side of the appliance exposed twice a day to the peptide T1 solution contained the peptide at a level of at least 0.4% w/wet wt of plaque and the level of calcium phosphate had increased 2-4 fold.

This work shows that peptide T1 is incorporated into plaque thereby increasing the plaque level of calcium and phosphate so inhibiting the caries process. This method of incorporation and accumulation in dental plaque can be used to carry remineralising and antibacterial ions into plaque and enamel e.g. Ca, PO₄, FPO₃, Zn, Cu, Sn, Ag, Al, Fe and La.

### Test 3 - Intra-Oral Remineralisation

An intra-oral appliance similar to that used in the previous test procedure was used except that the enamel slabs had been previously exposed to a demineralising solution to produce two sub-surface demineralised lesions in each slab. The demineralising solution was a 0.1M lactate buffer pH 5.0 containing 500 mg/L hydroxyapatite and 1% agar. The appliances ware worn by subjects for 10 days. Twice each day the appliances were removed and a drop of remineralising solution was placed on the enamel slabs on the right of the appliance. The left-side enamel slabs served as controls. After 10 days the enamel slabs were removed, sectioned and subjected to microradiography. The amount of mineral deposited back into the sub-surface lesions was determined using microdensitometry. The remineralising solution containing 1.8% w/v calcium phosphate T1 pH 7.0 returned 57% of the mineral lost compared with 13% by saliva alone.

### Test 4 - Plaque pH Fall

Subjects refrained from oral hygiene for 3-5 days than rinsed with a 5% sucrose solution for 1 min. Plaque samples were removed and pH was measured using the one drop technique. After approximaely 5 min the pH fell to around 5.0. However, if the subjects rinsed with a solution containing 1.8% w/v calcium phosphate T1, pH 7.0 15 min before rinsing with the 5% sucross solution the plaque pH did not fall below 6.7, demonstrating significant pH buffering by the calcium phosphate T1.

While the precise mechanism by which the phosphopeptides exhibit anticariogenic activity is not known, the following speculative theories have been put forward but are not to be taken as binding or limiting.

The phosphopeptides may accumulate in plaque and enamel, buffer plaque acid, prevent enamel demineralisation and enhance remineralisation. The small molecular weight of the phosphopeptides may allow penetration and accumulation is plaque and enamel pores. The phosphopeptides, due to the appropriate spacing of serine phosphate residues, may bind to tooth enamel mineral and prevent demineralisation. The peptides may also carry calcium and phosphate (fluorophosphate on modification) into plaque and enamel, in an appropriate form, possibly allowing spontaneous remineralisation. The phosphoserine residues may also buffer plaque acid. The phosphopeptide may also carry antibacterial metal ions e.g. Zn, Cu, Sn, Ag, Al, Fe and La into plaque and in this way have an antiplaque and antigingivitis affect. The metal ions are carried by the phosphopeptides primarily dine to the phosphoserine residues. Phosphopeptides may bind to plaque bacteria and inhibit sugar utilisation.

The ability of these peptides to sequester calcium phosphate can be utilised in the treatment of various rarefying bone diseases. These peptides can significantly increase the absorption of calcium, phosphate and iron in the gut. Hence, pharmaceutical vehicles (e.g. enteric capsules) or foods containing calcium phosphate T1 and ferrous phosphate T1 can be used for the treatment of osteoporosis/osteomalacia and anaemia.

Applicants have formulated various compositions in accordance with this invention as follows. In general, the compositions contain from 0.01-10% by weight of phosphopeptide.
Example 1. Flour: In a device for mixing dry substances, 1% by weight of calcium phosphate T1 was blended with flour.
Example 2. Cereal: A breakfast cereal was sprayed with a solution of calcium phosphate T1 in water. The cereal flakes were then dried to produce a finish ad product containing 1% calcium phosphate T1.
Example 3. Bread: 1% by weight of calcium phosphate T1 was added to the flour during the mixing of ingredients for the manufacture of bread.
Example 4. Cake mix: 1% by weight of calcium phosphate T1 was added to the dry ingredients in the preparation of a cake mix.
Example 5. Confectionary: In the preparation of confectionary 1% by weight of calcium phosphate T1 was added to the final mixture.
Example 6. Biscuit: In the preparation of a biscuit/mixture 1% by weight of calcium phosphate T1 was added to the other dry ingredients during mixing.
Example 7. Beverage: A beverage was prepared in which 0.1% weight of calcium phosphate T1 had been dissolved.
Example 8. Tablet: A tablet was made containing 10% by weight of calcium phosphate T1 together with excipients being flavouring matter and binding material.

In preparation of a typical dentifrice within the scope of this invention, the requisite salt and salts of the selected phosphopeptide are incorporated into dentifrice compositions in any suitable manner depending on whether a powder, paste or liquid preparation is to be produced. For this purpose appropriate preparations of surface-active agents, binders, flavouring materials and other excipients required to achieve the required form of dentifrice are added.

The invention is further illustrated by the following examples:
Example 9. Tooth paste: A toothpaste was prepared having the following composition:

| | |
|---|---|
| Calcium phosphate T1 | 5.0% by weight |
| CMC 7MF | 1.0% by weight |
| Saccharin 450 | 0.2% by weight |
| Glycerin (B.P.) | 25.0% by weight |
| Sodium lauryl sulphate (Empicol 0919) | 5.0% by weight |
| Sodium benzoate | 0.5% by weight |
| Flavour 9/693090 | 0.8% by weight |
| Calcium phosphate | 1.0% by weight |
| Water Deionised | 39.5% by weight |
| Thixosyl 33J | 9.5% by weight |
| Syloid AL-1 | 12.0% by weight |
| Titanium Dioxide 3328 | 0.5% by weight |

Example 10. Toothpaste: A preparation as set out in Example 9 was repeated but with the addition of 0.2% sodium fluoride in a suitable form.
Example 11. Toothpaste: A preparation as set out in Example 9 was repeated but with the addition of 0.4% stannous fluoride in a suitable form.
Example 12. Toothpaste: A preparation as set out in Example 9 was repeated but with the addition of 0.76% monosodium fluorophosphate in a suitable form.
Example 13. Toothpowder: The following preparation was made:

| | |
|---|---|
| Calcium phosphate T1 | 5.0% by weight |
| Soluble saccharin | 0.1% by weight |
| Colour agent | Trace |
| Dibasic calcium phosphate | 94.1% by weight |

Example 14. Toothpowder: A preparation as set out in Example 13 was made but with the addition of 0.76% monosodium fluorophosphate in a suitable form.
Example 15. Liquid dentifrice: A preparation was made consisting of:

| | |
|---|---|
| Sodium alginate | 1.4% by weight |
| Calcium phosphate T1 | 2.0% by weight |
| Sodium lauryl sulphate | 1.0% by weight |
| Flavouring | Trace |
| Colouring | Trace |
| Water | 95.5% by weight |

Example 16. Liquid dentifrice: As for Example 15 but with 0.5% sodium fluoride added.
Example 17. Mouthwash: The following preparation was made:

| | |
|---|---|
| Calcium phosphate T1 | 2.0% by weight |
| Sodium fluoride | 0.5% by weight |
| Flavouring | Trace |
| Colouring | Trace |
| Water | 97.5% by weight |

Example 18. Carbonated beverage: 0.1% by weight of calcium phosphopeptide T1 was added to a commercially available carbonated beverage.
Example 19. Fruit juice: 0.1% by weight of calcium phosphopeptide T1 was added to a commercially available fruit juice.
Example 20. Solution for topical application to teeth.

| | |
|---|---|
| Calcium Phosphate T1 | 2% |
| NaF | 0.6 mM |
| ZnAcetate | 0.1 mM |
| SrCl₂ | 0.1 mM |

(this solution may be formed into gel by increasing the amount of calcium phosphate T1).
Example 21. Dental filling material

| | |
|---|---|
| Calcium phosphate T1 | 5% w/w |
| Calcium phosphate | 95% w/w |
| Polymeriser | trace |

Made as a paste with water
The polymeriser used in this example was glutaraldehyde.
Example 22. Dental filling material.

| | |
|---|---|
| Calcium phosphate T1 | 5% w/w |
| Calcium phosphate | 70% |
| Acrylic polymer | 25% |
| Catalyst for polymer | trace |

Example 23. Topical Gel for the Treatment of hypersensitive teeth.

| | |
|---|---|
| Calcium phosphate T1 | 4.0% by weight |
| SrF₂ | 1.0% by weight |
| Flavouring | Trace |
| Water | 95% |

In the above calcium phosphate T1 was used for illustration but in lieu any appropriate phosphopeptide and/or salt might be used.

## Claims (Claims for the following Contracting State(s): BE, CH, LI, DE, FR, GB, IT, LU, NL, SE)

1. A composition for use in the inhibition of caries, comprising one or more phosphopeptides selected from:

T1: Glu-Met-Glu-Ala-Glu-Pse-Ile-Pse-Pse-Pse-Glu-Glu-
Ile-Val-Pro-Asn-Pse-Val-Glu-Gln-Lys;
T2: Glu-Leu-Glu-Glu-Leu-Asn-Val-Pro-Gly-Glu-Ile-Val-
Glu-Pse- Leu-Pse-Pse-Pse-Glu-Glu-Ser-Ile-Thr-Arg;
T3: Asn-Thr-Met-Glu-His-Val-Pse-Pse-Pse-Glu-Glu-Ser-
Ile-Ile- Pse-Gln-Glu-Thr-Tyr-Lys;
T4: Asn-Ala-Asn-Glu-Glu-Glu-Tyr-Ser-Ile-Gly-Pse-Pse-
Pse-Glu-Glu-Pse-Ala-Glu-Val-Ala-Thr-Glu-Glu-Val-Lys; and
T5: Glu-Gln-Leu-Pse-Pth-Pse-Glu-Glu-Asn-Ser-Lys.

2. A composition having anticariogenic activity comprising one or more phosphopeptides selected from: 
T1: Glu-Met-Glu-Ala-Glu-Pse-Ile-Pse-Pse-Pse-Glu-Glu-
Ile-Val-Pro-Asn-Pse-Val-Glu-Gln-Lys;
T2: Glu-Leu-Glu-Glu-Leu-Asn-Val-Pro-Gly-Glu-Ile-Val-
Glu-Pse- Leu-Pse-Pse-Pse-Glu-Glu-Ser-Ile-Thr-Arg;
T3: Asn-Thr-Met-Glu-His-Val-Pse-Pse-Pse-Glu-Glu-Ser-
Ile-Ile- Pse-Gln-Glu-Thr-Tyr-Lys;
T4: Asn-Ala-Asn-Glu-Glu-Glu-Tyr-Ser-Ile-Gly-Pse-Pse-
Pse-Glu-Glu-Pse-Ala-Glu-Val-Ala-Thr-Glu-Glu-Val-Lys; and
T5: Glu-Gln-Leu-Pse-Pth-Pse-Glu-Glu-Asn-Ser-Lys
 in the form of an acid or a salt, but not including a composition being a total tryptic digest of casein, with the proviso that where the phosphopeptide comprises T1 alone, the composition does not comprise the sequence:

Asp-Ile-Gly-Pse-Glu-Pse-Thr-Glu-Asp-Gln-Ala-Met-Glu-
Asp-Ile-Lys.

3. A composition according to Claim 2, comprising the peptide: 
T1: Glu-Met-Glu-Ala-Glu-Pse-Ile-Pse-Pse-Pse-Glu-Glu-
Ile-Val-Pro-Asn-Pse-Val-Glu-Gln-Lys;
 and one or more of the phosphopeptides:

T2: Glu-Leu-Glu-Glu-Leu-Asn-Val-Pro-Gly-Glu-Ile-Val-
Glu-Pse- Leu-Pse-Pse-Pse-Glu-Glu-Ser-Ile-Thr-Arg;
T3: Asn-Thr-Met-Glu-His-Val-Pse-Pse-Pse-Glu-Glu-Ser-
Ile-Ile- Pse-Gln-Glu-Thr-Tyr-Lys;
T4: Asn-Ala-Asn-Glu-Glu-Glu-Tyr-Ser-Ile-Gly-Pse-Pse-
Pse-Glu-Glu-Pse-Ala-Glu-Val-Ala-Thr-Glu-Glu-Val-Lys; and
T5: Glu-Gln-Leu-Pse-Pth-Pse-Glu-Glu-Asn-Ser-Lys.

4. The composition according to any preceding claim, further comprising a divalent metal salt.

5. The composition according to any preceding claim, further comprising a calcium phosphate salt.

6. The composition of Claim 5 comprising about 16 mole CaHPO₄ per mole of phosphopeptide(s).

7. The composition of Claim 1 or Claim 2, wherein the or each phosphopeptide is in the form of a sodium salt.

8. The composition of Claim 1 or Claim 2, further comprising hydroxyapatite.

9. The composition of any preceding claim, wherein the or each phosphopeptide is in substantially pure form.

10. The composition of any preceding claim, wherein the or each phosphopeptide is derived from casein, α-casein, β_ casein or a salt thereof.

11. The composition of any preceding claim, further comprising a physiological acceptable diluent.

12. The composition as claimed in Claim 11 in which the diluent is a pharmaceutically acceptable diluent, an orally ingestible material or a comestible.

13. The composition as claimed in any preceding claim, further comprising one or more remineralising and antibacterial ions.

14. The composition as claimed in Claim 13, in which the ions are selected from Ca, PO₄, FPO₃ Zn, Cu, Sn, Ag, Al, Fe and La.

15. The composition as claimed in any preceding claim substantially free of unpalatable impurities.

16. The composition as claimed in any preceding claim in which the phosphopeptide(s) is present in an amount from 0.01 to 10% by weight.

17. The composition as claimed in Claim 16 in which the phosphopeptide(s) is present in an amount from 0.01 to 5% by weight.

18. The composition as claimed in Claim 17 in which the phosphopeptide(s) is present in an amount from 0.01 to 2% by weight.

19. The composition as claimed in any preceding claim in the form of a foodstuff or confection; a toothpaste, toothpowder, dentifrice, mouthwash, dental filling composition or a preparation for topical application to teeth or gingival tissue; a solution or a gel.

## Claims (Claims for the following Contracting State(s): AT)

1. A method of preparing a composition for use in the inhibition of caries, comprising taking one or more phosphopeptides selected from: 
T1: Glu-Met-Glu-Ala-Glu-Pse-Ile-Pse-Pse-Pse-Glu-Glu-
Ile-Val-Pro-Asn-Pse-Val-Glu-Gln-Lys;
T2: Glu-Leu-Glu-Glu-Leu-Asn-Val-Pro-Gly-Glu-Ile-Val-
Glu-Pse- Leu-Pse-Pse-Pse-Glu-Glu-Ser-Ile-Thr-Arg;
T3: Asn-Thr-Met-Glu-His-Val-Pse-Pse-Pse-Glu-Glu-Ser-
Ile-Ile- Pse-Gln-Glu-Thr-Tyr-Lys;
T4: Asn-Ala-Asn-Glu-Glu-Glu-Tyr-Ser-Ile-Gly-Pse-Pse-
Pse-Glu-Glu-Pse-Ala-Glu-Val-Ala-Thr-Glu-Glu-Val-Lys; and
T5: Glu-Gln-Leu-Pse-Pth-Pse-Glu-Glu-Asn-Ser-Lys;
 and mixing said polypeptide in a physiologically acceptable diluent

2. A method of preparing a composition having anticariogenic activity comprising taking one or more phosphopeptides selected from: 
T1: Glu-Met-Glu-Ala-Glu-Pse-Ile-Pse-Pse-Pse-Glu-Glu-
Ile-Val-Pro-Asn-Pse-Val-Glu-Gln-Lys;
T2: Glu-Leu-Glu-Glu-Leu-Asn-Val-Pro-Gly-Glu-Ile-Val-
Glu-Pse- Leu-Pse-Pse-Pse-Glu-Glu-Ser-Ile-Thr-Arg;
T3: Asn-Thr-Met-Glu-His-Val-Pse-Pse-Pse-Glu-Glu-Ser-
Ile-Ile- Pse-Gln-Glu-Thr-Tyr-Lys;
T4: Asn-Ala-Asn-Glu-Glu-Glu-Tyr-Ser-Ile-Gly-Pse-Pse-
Pse-Glu-Glu-Pse-Ala-Glu-Val-Ala-Thr-Glu-Glu-Val-Lys; and
T5: Glu-Gln-Leu-Pse-Pth-Pse-Glu-Glu-Asn-Ser-Lys
 in the form of an acid or a salt, but not including a composition being a total tryptic digest of casein, with the proviso that where the phosphopeptide comprises T1 alone, the composition does not comprise the sequence: 
Asp-Ile-Gly-Pse-Glu-Pse-Thr-Glu-Asp-Gln-Ala-Met-Glu-
Asp-Ile-Lys;
 and mixing said one or more phosphopeptides with a physiologically acceptable divalent.

3. A method according to Claim 2, wherein said step of taking one or more phosphopeptides comprises taking the peptide: 
T1: Glu-Met-Glu-Ala-Glu-Pse-Ile-Pse-Pse-Pse-Glu-Glu-
Ile-Val-Pro-Asn-Pse-Val-Glu-Gln-Lys;
 and one or more of the phosphopeptides:

T2: Glu-Leu-Glu-Glu-Leu-Asn-Val-Pro-Gly-Glu-Ile-Val-
Glu-Pse- Leu-Pse-Pse-Pse-Glu-Glu-Ser-Ile-Thr-Arg;
T3: Asn-Thr-Met-Glu-His-Val-Pse-Pse-Pse-Glu-Glu-Ser-
Ile-Ile- Pse-Gln-Glu-Thr-Tyr-Lys;
T4: Asn-Ala-Asn-Glu-Glu-Glu-Tyr-Ser-Ile-Gly-Pse-Pse-
Pse-Glu-Glu-Pse-Ala-Glu-Val-Ala-Thr-Glu-Glu-Val-Lys; and
T5: Glu-Gln-Leu-Pse-Pth-Pse-Glu-Glu-Asn-Ser-Lys

4. The method according to any preceding claim, wherein said composition further comprises a divalent metal salt.

5. The method according to any preceding claim, wherein said composition further comprises a calcium phosphate salt.

6. The method of Claim 5 wherein said composition comprises about 16 mole CaHPO₄ per mole of phosphopeptide(s).

7. The method of Claim 1 or Claim 2, wherein the or each phosphopeptide is in the form of a sodium salt.

8. The method of Claim 1 or Claim 2, wherein said composition further comprises hydroxyapatite.

9. The method of any preceding claim, wherein the or each phosphopeptide is in substantially pure form.

10. The method of any preceding claim, wherein the or each phosphopeptide is derived from casein, α-casein, β_casein or a salt thereof.

11. The method as claimed in any preceding claim in which the diluent is a pharmaceutically acceptable diluent, an orally ingestible material or a comestible.

12. The method as claimed in any preceding claim, wherein the composition further comprises one or more remineralising and antibacterial ions.

13. The method as claimed in Claim 12, in which the ions are selected from Ca, PO₄, FPO₃ Zn, Cu, Sn, Ag, Al, Fe and La.

14. The method as claimed in any preceding claim wherein said composition is substantially free of unpalatable impurities.

15. The method as claimed in any preceding claim in which the phosphopeptide(s) is present in an amount from 0.01 to 10% by weight.

16. The method as claimed in Claim 15 in which the phosphopeptide(s) is present in an amount from 0.01 to 5% by weight.

17. The composition as claimed in Claim 16 in which the phosphopeptide(s) is present in an amount from 0.01 to 2% by weight.

18. The method as claimed in any preceding claim in the preparation of a foodstuff or confection; a toothpaste, toothpowder, dentifrice, mouthwash, dental filling composition or a preparation for topical application to teeth or gingival tissue; a solution or a gel.

## Patentansprüche (Patentansprüche für folgende(n) Vertragsstaat(en): BE, CH, LI, DE, FR, GB, IT, LU, NL, SE)

1. Eine Masse zur Verwendung bei der Hemmung von Karies, umfassend ein oder mehrere Phosphopeptide ausgewählt von:

T1: Glu-Met-Glu-Ala-Glu-Pse-Ile-Pse-Pse-Pse-Glu-
Glu-Ile-Val-Pro-Asn-Pse-Val-Glu-Gln-Lys;
T2: Glu-Leu-Glu-Glu-Leu-Asn-Val-Pro-Gly-Glu-Ile-
Val-Glu-Pse-Leu-Pse-Pse-Pse-Glu-Glu-Ser-Ile-Thr-Arg;
T3: Asn-Thr-Met-Glu-His-Val-Pse-Pse-Pse-Glu-Glu-
Ser-Ile-Ile-Pse-Gln-Glu-Thr-Tyr-Lys;
T4: Asn-Ala-Asn-Glu-Glu-Glu-Tyr-Ser-Ile-Gly-Pse-
Pse-Pse-Glu-Glu-Pse-Ala-Glu-Val-Ala-Thr-Glu-Glu-Val-Lys;
und
T5: Glu-Gln-Leu-Pse-Pth-Pse-Glu-Glu-Asn-Ser-Lys.

2. Eine Masse mit karieshemmender Wirkung, umfassend ein oder mehrere Phosphopeptide ausgewählt von: 
T1: Glu-Met-Glu-Ala-Glu-Pse-Ile-Pse-Pse-Pse-Glu-
Glu-Ile-Val-Pro-Asn-Pse-Val-Glu-Gln-Lys:
T2: Glu-Leu-Glu-Glu-Leu-Asn-Val-Pro-Gly-Glu-Ile-
Val-Glu-Pse-Leu-Pse-Pse-Pse-Glu-Glu-Ser-Ile-Thr-Arg;
T3: Asn-Thr-Met-Glu-His-Val-Pse-Pse-Pse-Glu-Glu-
Ser-Ile-Ile-Pse-Gln-Glu-Thr-Tyr-Lys;
T4: Asn-Ala-Asn-Glu-Glu-Glu-Tyr-Ser-Ile-Gly-Pse-
Pse-Pse-Glu-Glu-Pse-Ala-Glu-Val-Ala-Thr-Glu-Glu-Val-Lys;
und
T5: Glu-Gln-Leu-Pse-Pth-Pse-Glu-Glu-Asn-Ser-Lys
 in der Form einer Säure oder eines Salzes aber nicht einschließlich einer Masse, die ein vollständiges tryptisches Abbauprodukt von Casein ist, unter der Voraussetzung, daß die Masse, falls das Phosphopeptid nur T1 umfaßt, nicht die Folge:

Asp-Ile-Gly-Pse-Glu-Pse-Thr-Glu-Asp-Gln-Ala-Met-
Glu-Asp-Ile-Lys umfaßt.

3. Eine Masse nach Anspruch 2, umfassend das Peptid: 
T1: Glu-Met-Glu-Ala-Glu-Pse-Ile-Pse-Pse-Pse-Glu-
Glu-Ile-Val-Pro-Asn-Pse-Val-Glu-Gln-Lys
 sowie eines oder mehrere der Phosphopeptide:

T2: Glu-Leu-Glu-Glu-Leu-Asn-Val-Pro-Gly-Glu-Ile-
Val-Glu-Pse-Leu-Pse-Pse-Pse-Glu-Glu-Ser-Ile-Thr-Arg;
T3: Asn-Thr-Met-Glu-His-Val-Pse-Pse-Pse-Glu-Glu-
Ser-Ile-Ile-Pse-Gln-Glu-Thr-Tyr-Lys;
T4: Asn-Ala-Asn-Glu-Glu-Glu-Tyr-Ser-Ile-Gly-Pse-
Pse-Pse-Glu-Glu-Pse-Ala-Glu-Val-Ala-Thr-Glu-Glu-Val-Lys;
und
T5: Glu-Gln-Leu-Pse-Pth-Pse-Glu-Glu-Asn-Ser-Lys.

4. eine Masse nach einem der vorstehenden Ansprüche, wobei diese des weiteren ein zweiwertiges Metallsalz umfaßt.

5. eine Masse nach einem der vorstehenden Ansprüche, wobei diese des weiteren ein Calciumphosphatsalz umfaßt.

6. Die Masse nach Anspruch 5, wobei diese etwa 16 Mol CaHPO₄ je Mol Phosphopeptid(e) umfaßt.

7. Die Masse nach Anspruch 1 oder Anspruch 2, wobei das bzw. jedes Phosphopeptid in der Form eines Natriumsalzes vorliegt.

8. Die Masse nach Anspruch 1 oder Anspruch 2, wobei diese des weiteren Hydroxyapatit umfaßt.

9. Die Masse nach einem der vorstehenden Ansprüche, wobei das bzw. jedes Phosphopeptid in im wesentlichen reiner Form vorliegt.

10. Die Masse nach einem der vorstehenden Ansprüche, wobei das bzw. jedes Phosphopeptid von Casein, α-Casein, β-Casein oder einem Salz davon deriviert ist.

11. Die Masse nach einem der vorstehenden Ansprüche, wobei diese des weiteren ein physiologisch annehmbares Verdünnungsmittel umfaßt.

12. Die Masse nach Anspruch 11, wobei das Verdünnungsmittel ein pharmazeutisch annehmbares Verdünnungsmittel, ein oral einnehmbares Material oder ein verzehrbarer Stoff ist.

13. Die Masse nach einem der vorstehenden Ansprüche, wobei diese des weiteren ein oder mehrere remineralisierende und antibakterielle Ionen umfaßt.

14. Die Masse nach Anspruch 13, wobei die Ionen aus der Gruppe Ca, PO₄, FPO₃, Zn, Cu, Sn, Ag, Al, Fe und La ausgewählt sind.

15. Die Masse nach einem der vorstehenden Ansprüche, wobei diese im wesentlichen von schlecht schmeckenden Verunreinigungen frei ist.

16. Die Masse nach einem der vorstehenden Ansprüche, wobei das Phosphopeptid (die Phosphopeptide) in einem Gewichtsanteil von 0,01 bis 10 % vorhanden ist (sind).

17. Die Masse nach Anspruch 16, wobei das Phosphopeptid (die Phosphopeptide) in einem Gewichtsanteil von 0,01 bis 5 % vorhanden ist (sind).

18. Die Masse nach Anspruch 17, wobei das Phosphopeptid (die Phosphopeptide) in einem Gewichtsanteil von 0,01 bis 2 % vorhanden ist (sind).

19. Die Masse nach einem der vorstehenden Ansprüche in der Form eines Lebensmittels oder Konfekts; einer Zahnpaste, eines Zahnpulvers, eines Zahnputzmittels, eines Mundwassers, einer Zahnfüllmasse oder eines Präparats für örtliche Anwendung bei Zähnen oder Zahnfleischgewebe, einer Lösung oder eines Gels.

## Patentansprüche (Patentansprüche für folgende(n) Vertragsstaat(en): AT)

1. Ein Verfahren zur Herstellung einer Masse zur Verwendung bei der Hemmung von Karies, umfassend den Gebrauch von einem oder mehreren Phosphopeptiden ausgewählt von: 
T1: Glu-Met-Glu-Ala-Glu-Pse-Ile-Pse-Pse-Pse-Glu-
Glu-Ile-Val-Pro-Asn-Pse-Val-Glu-Gln-Lys;
T2: Glu-Leu-Glu-Glu-Leu-Asn-Val-Pro-Gly-Glu-Ile-
Val-Glu-Pse-Leu-Pse-Pse-Pse-Glu-Glu-Ser-Ile-Thr-Arg;
T3: Asn-Thr-Met-Glu-His-Val-Pse-Pse-Pse-Glu-Glu-
Ser-Ile-Ile-Pse-Gln-Glu-Thr-Tyr-Lys;
T4: Asn-Ala-Asn-Glu-Glu-Glu-Tyr-Ser-Ile-Gly-Pse-
Pse-Pse-Glu-Glu-Pse-Ala-Glu-Val-Ala-Thr-Glu-Glu-Val-Lys;
und
T5: Glu-Gln-Leu-Pse-Pth-Pse-Glu-Glu-Asn-Ser-Lys;
 sowie Mischen des besagten Phosphopeptids in einem physiologisch annehmbaren Verdünnungsmittel.

2. Ein Verfahren zur Herstellung einer Masse mit karieshemmender Wirkung, umfassend den Gebrauch von einem oder mehreren Phosphopeptiden ausgewählt von: 
T1: Glu-Met-Glu-Ala-Glu-Pse-Ile-Pse-Pse-Pse-Glu-
Glu-Ile-Val-Pro-Asn-Pse-Val-Glu-Gln-Lys;
T2: Glu-Leu-Glu-Glu-Leu-Asn-Val-Pro-Gly-Glu-Ile-
Val-Glu-Pse-Leu-Pse-Pse-Pse-Glu-Glu-Ser-Ile-Thr-Arg;
T3: Asn-Thr-Met-Glu-His-Val-Pse-Pse-Pse-Glu-Glu-
Ser-Ile-Ile-Pse-Gln-Glu-Thr-Tyr-Lys;
T4: Asn-Ala-Asn-Glu-Glu-Glu-Tyr-Ser-Ile-Gly-Pse-
Pse-Pse-Glu-Glu-Pse-Ala-Glu-Val-Ala-Thr-Glu-Glu-Val-Lys;
und
T5: Glu-Gln-Leu-Pse-Pth-Pse-Glu-Glu-Asn-Ser-Lys
 in der Form einer Säure oder eines Salzes aber nicht einschließlich einer Masse, die ein vollständiges tryptisches Abbauprodukt von Casein ist, unter der Voraussetzung, daß die Masse, falls das Phosphopeptid nur T1 umfaßt, nicht die Folge: 
Asp-Ile-Gly-Pse-Glu-Pse-Thr-Glu-Asp-Gln-Ala-Met-
Glu-Asp-Ile-Lys umfaßt;
 sowie Mischen des besagten einen Phosphopeptids bzw. der besagten mehreren Phosphopeptide mit einem physiologisch annehmbaren zweiwertigen Stoff.

3. Ein Verfahren nach Anspruch 2, bei dem der besagte Schritt des Gebrauchs von einem bzw. mehreren Phosphopeptiden den Gebrauch des Peptids: 
T1: Glu-Met-Glu-Ala-Glu-Pse-Ile-Pse-Pse-Pse-Glu-
Glu-Ile-Val-Pro-Asn-Pse-Val-Glu-Gln-Lys;
 und eines oder mehrerer der Phosphopeptide:

T2: Glu-Leu-Glu-Glu-Leu-Asn-Val-Pro-Gly-Glu-Ile-
Val-Glu-Pse-Leu-Pse-Pse-Pse-Glu-Glu-Ser-Ile-Thr-Arg;
T3: Asn-Thr-Met-Glu-His-Val-Pse-Pse-Pse-Glu-Glu-
Ser-Ile-Ile-Pse-Gln-Glu-Thr-Tyr-Lys;
T4: Asn-Ala-Asn-Glu-Glu-Glu-Tyr-Ser-Ile-Gly-Pse-
Pse-Pse-Glu-Glu-Pse-Ala-Glu-Val-Ala-Thr-Glu-Glu-Val-Lys;
und
T5: Glu-Gln-Leu-Pse-Pth-Pse-Glu-Glu-Asn-Ser-Lys
umfaßt.

4. Das Verfahren nach einem der vorstehenden Ansprüche, wobei die besagte Masse des weiteren ein zweiwertiges Metallsalz umfaßt.

5. Das Verfahren nach einem der vorstehenden Ansprüche, wobei die besagte Masse des weiteren ein Calciumphosphatsalz umfaßt.

6. Das Verfahren nach Anspruch 5, wobei die besagte Masse etwa 16 Mol CaHPO₄ je Mol Phosphopeptid(e) umfaßt.

7. Das Verfahren nach Anspruch 1 oder Anspruch 2, wobei das bzw. jedes Phosphopeptid in der Form eines Natriumsalzes vorliegt.

8. Das Verfahren nach Anspruch 1 oder Anspruch 2, wobei die besagte Masse des weiteren Hydroxyapatit umfaßt.

9. Das Verfahren nach einem der vorstehenden Ansprüche, wobei das bzw. jedes Phosphopeptid in im wesentlichen reiner Form vorliegt.

10. Das Verfahren nach einem der vorstehenden Ansprüche, wobei das bzw. jedes Phosphopeptid von Casein, α-Casein, β-Casein oder einem Salz davon deriviert ist.

11. Das Verfahren nach einem der vorstehenden Ansprüche, wobei das Verdünnungsmittel ein pharmazeutisch annehmbares Verdünnungsmittel, ein oral einnehmbares Material oder ein verzehrbarer Stoff ist.

12. Das Verfahren nach einem der vorstehenden Ansprüche, wobei die Masse des weiteren ein oder mehrere remineralisierende und antibakterielle Ionen umfaßt.

13. Das Verfahren nach Anspruch 12, wobei die Ionen aus der Gruppe Ca, PO₄, FPO₃, Zn, Cu, Sn, Ag, Al, Fe und La ausgewählt sind.

14. Das Verfahren nach einem der vorstehenden Ansprüche, wobei die besagte Masse im wesentlichen von schlecht schmeckenden Verunreinigungen frei ist.

15. Das Verfahren nach einem der vorstehenden Ansprüche, wobei das Phosphopeptid (die Phosphopeptide) in einem Gewichtsanteil von 0,01 bis 10 % vorhanden ist (sind).

16. Das Verfahren nach Anspruch 15, wobei das Phosphopeptid (die Phosphopeptide) in einem Gewichtsanteil von 0,01 bis 5 % vorhanden ist (sind).

17. Das Verfahren nach Anspruch 16, wobei das Phosphopeptid (die Phosphopeptide) in einem Gewichtsanteil von 0,01 bis 2 % vorhanden ist (sind).

18. Das Verfahren nach einem der vorstehenden Ansprüche betreffend die Herstellung eines Lebensmittels oder Konfekts; einer Zahnpaste, eines Zahnpulvers, eines Zahnputzmittels, eines Mundwassers, einer Zahnfüllmasse oder eines Präparats für örtliche Anwendung auf Zähne oder Zahnfleischgewebe; einer Lösung oder eines Gels.

## Revendications (Revendications pour l'(les) Etat(s) contractant(s) suivant(s): BE, CH, LI, DE, FR, GB, IT, LU, NL, SE)

1. Composition pour utilisation dans l'inhibition des caries, comprenant un ou plusieurs phosphopeptides sélectionnés parmi :

T1 : Glu-Met-Glu-Ala-Glu-Pse-Ile-Pse-Pse-Pse-Glu-Glu-
Ile-Val-Pro-Asn-Pse-Val-Glu-Gln-Lys ;
T2 : Glu-Leu-Glu-Glu-Leu-Asn-Val-Pro-Gly-Glu-Ile-Val-
Glu-Pse-Leu-Pse-Pse-Pse-Glu-Glu-Ser-Ile-Thr-Arg ;
T3 : Asn-Thr-Met-Glu-His-Val-Pse-Pse-Pse-Glu-Glu-Ser-
Ile-Ile-Pse-Gln-Glu-Thr-Tyr-Lys ;
T4 : Asn-Ala-Asn-Glu-Glu-Glu-Tyr-Ser-Ile-Gly-Pse-Pse-
Pse-Glu-Glu-Pse-Ala-Glu-Val-Ala-Thr-Glu-Glu-Val-Lys ; et
T5 : Glu-Gln-Leu-Pse-Pth-Pse-Glu-Glu-Asn-Ser-Lys.

2. Composition ayant une activité anticariogénique, comprenant un ou plusieurs phosphopeptides sélectionnés parmi : 
T1 : Glu-Met-Glu-Ala-Glu-Pse-Ile-Pse-Pse-Pse-Glu-Glu-
Ile-Val-Pro-Asn-Pse-Val-Glu-Gln-Lys ;
T2 : Glu-Leu-Glu-Glu-Leu-Asn-Val-Pro-Gly-Glu-Ile-Val-
Glu-Pse-Leu-Pse-Pse-Pse-Glu-Glu-Ser-Ile-Thr-Arg ;
T3 : Asn-Thr-Met-Glu-His-Val-Pse-Pse-Pse-Glu-Glu-Ser-
Ile-Ile-Pse-Gln-Glu-Thr-Tyr-Lys ;
T4 : Asn-Ala-Asn-Glu-Glu-Glu-Tyr-Ser-Ile-Gly-Pse-Pse-
Pse-Glu-Glu-Pse-Ala-Glu-Val-Ala-Thr-Glu-Glu-Val-Lys ; et
T5 : Glu-Gln-Leu-Pse-Pth-Pse-Glu-Glu-Asn-Ser-Lys
 sous la forme d'un acide ou d'un sel, mais ne comprenant pas une composition qui est un digeste tryptique total de la caséine, étant entendu que lorsque le phosphopeptide comprend T1 seul, la composition ne comprend pas la séquence:

Asp-Ile-Gly-Pse-Glu-Pse-Thr-Glu-Asp-Gln-Ala-Met-Glu-
Asp-Ile-Lys.

3. Composition selon la Revendication 2, comprenant le peptide : 
T1 : Glu-Met-Glu-Ala-Glu-Pse-Ile-Pse-Pse-Pse-Glu-Glu-
Ile-Val-Pro-Asn-Pse-Val-Glu-Gln-Lys ;
 et un ou plusieurs des phosphopeptides :

T2 : Glu-Leu-Glu-Glu-Leu-Asn-Val-Pro-Gly-Glu-Ile-Val-
Glu-Pse-Leu-Pse-Pse-Pse-Glu-Glu-Ser-Ile-Thr-Arg ;
T3 : Asn-Thr-Met-Glu-His-Val-Pse-Pse-Pse-Glu-Glu-Ser-
Ile-Ile-Pse-Gln-Glu-Thr-Tyr-Lys ;
T4 : Asn-Ala-Asn-Glu-Glu-Glu-Tyr-Ser-Ile-Gly-Pse-Pse-
Pse-Glu-Glu-Pse-Ala-Glu-Val-Ala-Thr-Glu-Glu-Val-Lys ; et
T5 : Glu-Gln-Leu-Pse-Pth-Pse-Glu-Glu-Asn-Ser-Lys.

4. Composition selon l'une quelconque des revendications précédentes, comprenant en outre un sel de métal bivalent.

5. Composition selon l'une quelconque des revendications précédentes, comprenant en outre un sel de phosphate de calcium.

6. Composition selon la Revendication 5 comprenant environ 16 moles de CaHPO₄ par mole de phosphopeptide(s).

7. Composition selon la Revendication 1 ou la Revendication 2, dans laquelle le phosphopeptide ou chacun d'eux est sous la forme d'un sel de sodium.

8. Composition selon la Revendication 1 ou la Revendication 2, comprenant en outre de l'hydroxyapatite.

9. Composition selon l'une quelconque des revendications précédentes, dans laquelle le phosphopeptide ou chacun d'eux est sous une forme substantiellement pure.

10. Composition selon l'une quelconque des revendications précédentes, dans laquelle le phosphopeptide ou chacun d'eux est dérivé de la caséine, α-caséine, β-caséine ou d'un sel de ces dernières.

11. Composition selon l'une quelconque des revendications précédentes, comprenant en outre un diluant physiologique acceptable.

12. Composition selon la Revendication 11, dans laquelle le diluant et un diluant pharmaceutiquement acceptable, une matière pouvant être ingérée oralement ou un produit comestible.

13. Composition selon l'une quelconque des revendications précédentes, comprenant en outre un ou plusieurs ions reminéralisants et antibactériens.

14. Composition selon la Revendication 13, dans laquelle les ions sont sélectionnés parmi Ca, PO₄, FPO₃ Zn, Cu, Sn, Ag, Al, Fe et La.

15. Composition selon l'une quelconque des revendications précédentes, substantiellement exempte d'impuretés désagréables au goût.

16. Composition selon l'une quelconque des revendications précédentes, dans laquelle le ou les phosphopeptides sont présents à raison de 0,01 à 10% en poids.

17. Composition selon la Revendication 16 dans laquelle le ou les phosphopeptides sont présents à raison de 0,01 à 5% en poids.

18. Composition selon la Revendication 17, dans laquelle le ou les phosphopeptides sont présents à raison de 0,01 à 2% en poids.

19. Composition selon l'une quelconque des revendications précédentes, sous la forme d'aliment ou de friandise ; de pâte dentaire, poudre dentaire, dentifrice, bain de bouche, composition pour obturation dentaire, ou de préparation pour application topique sur les dents ou le tissu gingival ; de solution ou de gel.

## Revendications (Revendications pour l'(les) Etat(s) contractant(s) suivant(s): AT)

1. Procédé de préparation d'une composition pour utilisation dans l'inhibition des caries, comprenant la prise d'un ou plusieurs phosphopeptides sélectionnés parmi : 
T1 : Glu-Met-Glu-Ala-Glu-Pse-Ile-Pse-Pse-Pse-Glu-Glu-
Ile-Val-Pro-Asn-Pse-Val-Glu-Gln-Lys ;
T2 : Glu-Leu-Glu-Glu-Leu-Asn-Val-Pro-Gly-Glu-Ile-Val-
Glu-Pse-Leu-Pse-Pse-Pse-Glu-Glu-Ser-Ile-Thr-Arg ;
T3 : Asn-Thr-Met-Glu-His-Val-Pse-Pse-Pse-Glu-Glu-Ser-
Ile-Ile-Pse-Gln-Glu-Thr-Tyr-Lys ;
T4 : Asn-Ala-Asn-Glu-Glu-Glu-Tyr-Ser-Ile-Gly-Pse-Pse-
Pse-Glu-Glu-Pse-Ala-Glu-Val-Ala-Thr-Glu-Glu-Val-Lys ; et
T5 : Glu-Gln-Leu-Pse-Pth-Pse-Glu-Glu-Asn-Ser-Lys ;
 et le mélange dudit polypeptide dans un diluant physiologiquement acceptable.

2. Procédé de préparation d'une composition ayant une activité anticariogénique comprenant la prise d'un ou plusieurs phosphopeptides sélectionnés parmi : 
T1 : Glu-Met-Glu-Ala-Glu-Pse-Ile-Pse-Pse-Pse-Glu-Glu-
Ile-Val-Pro-Asn-Pse-Val-Glu-Gln-Lys ;
T2 : Glu-Leu-Glu-Glu-Leu-Asn-Val-Pro-Gly-Glu-Ile-Val-
Glu-Pse-Leu-Pse-Pse-Pse-Glu-Glu-Ser-Ile-Thr-Arg ;
T3 : Asn-Thr-Met-Glu-His-Val-Pse-Pse-Pse-Glu-Glu-Ser-
Ile-Ile-Pse-Gln-Glu-Thr-Tyr-Lys ;
T4 : Asn-Ala-Asn-Glu-Glu-Glu-Tyr-Ser-Ile-Gly-Pse-Pse-
Pse-Glu-Glu-Pse-Ala-Glu-Val-Ala-Thr-Glu-Glu-Val-Lys ; et
T5 : Glu-Gln-Leu-Pse-Pth-Pse-Glu-Glu-Asn-Ser-Lys
 sous la forme d'un acide ou d'un sel, mais ne comprenant pas une composition qui est un digeste tryptique total de la caséine, étant entendu que lorsque le phosphopeptide comprend T1 seul, la composition ne comprend pas la séquence: 
Asp-Ile-Gly-Pse-Glu-Pse-Thr-Glu-Asp-Gln-Ala-Met-Glu-
Asp-Ile-Lys ;
 et le mélange dudit ou desdits phosphopeptides avec un diluant physiologiquement acceptable.

3. Procédé selon la Revendication 2, dans lequel ladite étape de prise d'un ou plusieurs phosphopeptides comprend la prise du peptide : 
T1 : Glu-Met-Glu-Ala-Glu-Pse-Ile-Pse-Pse-Pse-Glu-Glu-
Ile-Val-Pro-Asn-Pse-Val-Glu-Gln-Lys ;
 et d'un ou plusieurs des phosphopeptides :

T2 : Glu-Leu-Glu-Glu-Leu-Asn-Val-Pro-Gly-Glu-Ile-Val-
Glu-Pse-Leu-Pse-Pse-Pse-Glu-Glu-Ser-Ile-Thr-Arg ;
T3 : Asn-Thr-Met-Glu-His-Val-Pse-Pse-Pse-Glu-Glu-Ser-
Ile-Ile-Pse-Gln-Glu-Thr-Tyr-Lys ;
T4 : Asn-Ala-Asn-Glu-Glu-Glu-Tyr-Ser-Ile-Gly-Pse-Pse-
Pse-Glu-Glu-Pse-Ala-Glu-Val-Ala-Thr-Glu-Glu-Val-Lys ; et
T5 : Glu-Gln-Leu-Pse-Pth-Pse-Glu-Glu-Asn-Ser-Lys.

4. Procédé selon l'une quelconque des revendications précédentes, dans lequel ladite composition comprend en outre un sel de métal bivalent.

5. Procédé selon l'une quelconque des revendications précédentes, dans lequel ladite composition comprend en outre un sel de phosphate de calcium.

6. Procédé selon la Revendication 5, dans lequel ladite composition comprend environ 16 moles de CaHPO₄ par mole de phosphopeptide(s).

7. Procédé selon la Revendication 1 ou la Revendication 2, dans lequel le phosphopeptide ou chacun d'eux est sous la forme d'un sel de sodium.

8. Procédé selon la Revendication 1 ou la Revendication 2, dans lequel ladite composition comprend en outre de l'hydroxyapatite.

9. Procédé selon l'une quelconque des revendications précédentes, dans lequel le phosphopeptide ou chacun d'eux est sous une forme substantiellement pure.

10. Procédé selon l'une quelconque des revendications précédentes, dans lequel le phosphopeptide ou chacun d'eux est dérivé de la caséine, α-caséine, β-caséine ou d'un sel de ces dernières.

11. Procédé selon l'une quelconque des revendications précédentes, dans lequel le diluant est un diluant pharmaceutiquement acceptable, une matière pouvant être ingérée oralement ou un produit comestible.

12. Procédé selon l'une quelconque des revendications précédentes, dans lequel la composition comprend en outre un ou plusieurs ions reminéralisants et antibactériens.

13. Procédé selon la Revendication 12, dans lequel les ions sont choisis parmi Ca, PO₄, FPO₃ Zn, Cu, Sn, Ag, Al, Fe et La.

14. Procédé selon l'une quelconque des revendications précédentes, dans lequel ladite composition est substantiellement exempte d'impuretés désagréables au goût.

15. Procédé selon l'une quelconque des revendications précédentes, dans lequel le ou les phosphopeptides sont présents à raison de 0,01 à 10% en poids.

16. Procédé selon la Revendication 15, dans lequel le ou les phosphopeptides sont présents à raison de 0,01 à 5% en poids.

17. Composition selon la Revendication 16 dans laquelle le ou les phosphopeptides sont présents à raison de 0,01 à 2% en poids.

18. Procédé selon l'une quelconque des revendications précédentes dans la préparation d'un aliment ou d'une friandise ; d'une pâte dentaire, d'une poudre dentaire, d'un dentifrice, d'un bain de bouche, d'une composition pour obturation dentaire, ou d'une préparation pour application topique sur les dents ou le tissu gingival ; d'une solution ou d'un gel.
